# EUROPEAN PATENT SPECIFICATION

(11) **EP 4 162 935 B1**
(45) Date of publication and mention of the grant of the patent: **20.08.2025**
(21) Application number: 22200887.2
(22) Date of filing: 11.10.2022
(51) Int. Cl.: A61K 31/352, A61P 31/14, A61K 36/63, A61K 36/752

(54) **DIOSMETIN FOR TREATMENT OF HUMAN CORONAVIRUS INFECTION**
DIOSMETIN ZUR BEHANDLUNG VON INFEKTIONEN DURCH DAS MENSCHLICHE CORONAVIRUS
DIOSMETINE POUR LE TRAITEMENT D'UNE INFECTION PAR LE CORONAVIRUS HUMAIN

(30) Priority: 11.10.2021 PT 2021117506
(43) Date of publication of application: 12.04.2023
(73) Proprietor: UNIVERSIDADE NOVA DE LISBOA, 1099-085 Lisboa (PT)
(72) Inventor: TEIXEIRA SARAMAGO, ANA MARGARIDA, 1675-168 PONTINHA (PT); GONÇALVES MATOS LUÍS, RUTE MARGARIDA, 2775-623 CARCAVELOS (PT); PAIS DE FARIA DE ANDRADE ARRAIANO, CECÍLIA MARIA, 1990-122 LISBOA (PT); SILVA SOUZA, CAIO, 2780-109 OEIRAS (PT); PEREIRA LOUSA, DIANA ANDREIA, 2775-800 CARCAVELOS (PT); SIMÕES LOUREIRO NUNES SOARES, CLÁUDIO MANUEL, 2775-800 CARCAVELOS (PT); SOUSA PINTO TORRES FEVEREIRO, MIGUEL AGOSTINHO, 1200-856 LISBOA (PT); FERREIRA HENRIQUES DE OLIVEIRA MOURÃO, ANA MARGARIDA, 2560-286 TORRES VEDRAS (PT)
(74) Representative: Schulz Junghans Patentanwälte PartGmbB

(56) References cited:
- WO-A1-2022/119548
- KHAN JISHAN ET AL: "Identification of potential phytochemicals from Citrus Limon against main protease of SARS-CoV-2: molecular docking, molecular dynamic simulations and quantum computations", JOURNAL OF BIOMOLECULAR STRUCTURE & DYNAMICS, vol. 40, no. 21, 19 July 2021 (2021-07-19), US, pages 10741 - 10752, XP093019837, ISSN: 0739-1102, DOI: 10.1080/07391102.2021.1947893
- KUMAR SUMIT ET AL: "Multi-targeting approach for nsp3, nsp9, nsp12 and nsp15 proteins of SARS-CoV-2 by Diosmin as illustrated by molecular docking and molecular dynamics simulation methodologies", METHODS, vol. 195, 25 February 2021 (2021-02-25), NL, pages 44 - 56, XP093021134, ISSN: 1046-2023, DOI: 10.1016/j.ymeth.2021.02.017
- LEE DAE-HYO ET AL: "Anti-inflammatory effects of natural flavonoid diosmetin in IL-4 and LPS-induced macrophage activation and atopic dermatitis model", INTERNATIONAL IMMUNOPHARMACOLOGY, ELSEVIER, AMSTERDAM, NL, vol. 89, 9 October 2020 (2020-10-09), XP086386570, ISSN: 1567-5769, [retrieved on 20201009], DOI: 10.1016/J.INTIMP.2020.107046
- RASHED KHALED ET AL: "IN-VITRO ANTIVIRAL ACTIVITY OF PISTACIA CHINENSIS FLAVONOIDS AGAINST HEPATITIS C VIRUS (HCV)", JOURNAL OF APPLIED PHARMACY, vol. 6, no. 1, 1 January 2014 (2014-01-01), pages 8 - 18, XP093020226, Retrieved from the Internet <URL:https://www.longdom.org/open-access-pdfs/invitro-antiviral-activity-of-pistacia-chinensis-flavonoids-against-hepatitis-c-virus-hcv-.pdf> DOI: 10.21065/19204159.6.8
- SARAMAGO MARGARIDA ET AL: "New targets for drug design: importance of nsp14/nsp10 complex formation for the 3'-5' exoribonucleolytic activity on SARS-CoV-2", THE FEBS JOURNAL, vol. 288, no. 17, 28 April 2021 (2021-04-28), GB, pages 5130 - 5147, XP093020061, ISSN: 1742-464X, Retrieved from the Internet <URL:https://onlinelibrary.wiley.com/doi/full-xml/10.1111/febs.15815> DOI: 10.1111/febs.15815
- WANG JIN ET AL: "Investigation into the in vivo mechanism of diosmetin in patients with breast cancer and COVID-19 using bioinformatics", FRONTIERS IN PHARMACOLOGY, vol. 13, 17 August 2022 (2022-08-17), XP093019777, DOI: 10.3389/fphar.2022.983821
- FAN LIMING ET AL: "Chemical composition and therapeutic mechanism of Xuanbai Chengqi Decoction in the treatment of COVID-19 by network pharmacology, molecular docking and molecular dynamic analysis", MOLECULAR DIVERSITY, 8 March 2022 (2022-03-08), Cham, XP093019829, ISSN: 1381-1991, Retrieved from the Internet <URL:https://link.springer.com/article/10.1007/s11030-022-10415-7/fulltext.html> DOI: 10.1007/s11030-022-10415-7

## Description

The present invention relates to the use of diosmetin for treatment of human coronavirus infection.

### Background of the Invention

The human coronavirus (hCoV) pandemic, caused by SARS-Coronavirus-2 (SARS-CoV-2), dramatically affects individuals and societies across the world. We currently lack a universally applied vaccine and effective anti-viral medication. Moreover, the longevity of the immunity provided by currently available vaccines as well as their efficiency in preventing infections with the novel strains of coronavirus remains unknown. The inhibition of early disease onset after onset of symptoms in individuals with high risk of a grave course of COVID-19 is key to protect lives and guard against overload of the health care systems. Currently, there is no effective clinical treatment against this disease. Only a small number of drugs are FDA approved for treatment of COVID-19, including Remdesivir and a small number of monoclonal antibodies, which are expensive to produce. To reduce COVID-19 mortality and disease burden on societies, anti-viral treatments and vaccination are urgently needed.

Furthermore, previous appearances of highly infectious coronaviruses in humans that are transmissible between human patients and lead to high mortality rates, such as SARS-CoV-1 and Middle East respiratory syndrome (MERS), show that it would be useful to have medication with a broad applicability to human coronavirus infection ready in the case of another unrelated emergence of a novel coronavirus that is infectious and transmissible in humans.

Diosmetin (5,7-Dihydroxy-2-(3-hydroxy-4-methoxyphenyl)-4H-benzopyran-4-one, CAS Number: 520-34-3) is an O-methylated flavone, and the aglycone part of the flavonoid glycosides diosmin, which occurs naturally in citrus species (Roowi et al 2011. J Agric Food Chem. 59: 12217-12225) and olive leaves (Olea europaea L.) (Meirinhos et al 2005. Nat Prod Res. 19: 189-195). It has been found to act as a weak TrkB receptor agonist. Pharmacologically, diosmetin is reported to exhibit anticancer, antimicrobial, antioxidant, oestrogenic and anti-inflammatory activities.

Based on the above-mentioned state of the art, the objective of the present invention is to provide means and methods to treat human coronavirus infections, including SARS-CoV-2, in human patients. This objective is attained by the subject-matter of the independent claims of the present specification, with further advantageous embodiments described in the dependent claims, examples, figures and general description of this specification. Any references in the description to methods of treatment refer to the compounds, pharmaceutical compositions and medicaments of the present invention for use in a method for treatment of the human (or animal) body by therapy (or for diagnosis).

### Summary of the Invention

The present invention relates to the drug diosmetin, 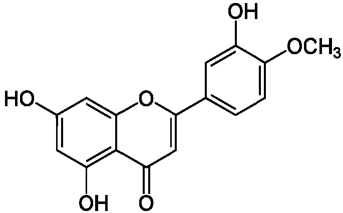 diosmetin for use in the treatment of infection of human coronavirus, particularly infection by SARS-CoV-2 and its variants (e.g. alpha, beta, gamma, delta, omicron and others).

Diosmetin has been found to inhibit the activity of two viral RNA nucleases: nonstructural protein 14 (nsp14) and nonstructural protein 15 (nsp15), both of which are crucial for the life cycle of coronaviruses. Nsp14 is an N-terminal 3'-5' exoribonuclease (ExoN) responsible for viral RNA proofreading avoiding lethal mutagenesis, and a C-terminal N7-methyltransferase (N7-MTase) involved in attaching a 5'-cap to the viral RNA, so that the viral RNA is able to use the host translation machinery and is not recognized as foreign. Nsp15 is a uridine-specific endoribonuclease responsible for destroying U-rich intermediate RNAs that function as activators of the host immune system. Both proteins are essential for virus amplification, are found exclusively in the genome of coronaviruses, and function as major interferon antagonists in SARS-CoV-2 (Yuen et al CK Emerg Microbes Infect. 2020, 9:1418-1428).

Diosmetin has been identified as a potential inhibitor of nsp14 and nsp15 in a docking screen of a library of 8368 drugs, either experimental or already approved for other conditions. These target proteins are highly conserved among coronaviruses. Thus, compounds found to have the ability of inhibiting SARS-CoV-2 RNases are expected to be active across the family *Coronaviridae,* extending their therapeutic potential to other clinically relevant coronaviruses (Yoshimoto et al 2020. Protein J. 39(3): 198-216; Saramago, M., et al. The FEBS Journal 2021, 288: 5130-5147; Saramago M., et al. Microorganisms 2022, 210(2):342022).

The enormous mutating ability of SARS-CoV-2 makes it more potent than previous coronavirus strains. Such variation capacity originates new strains with different infection patterns. Different mutant strains of this virus are constantly being identified in distinct parts of the world. The different variants of SARS-CoV-2 that have emerged are highly associated with multiple mutations in structural proteins, especially in the Spike protein. For these reasons, the present invention consists of a treatment against these highly conserved targets (nsp14 and nsp15).

Flavonoids have been studied against a wide range of DNA and RNA viruses (Badshah et al 2021 Biomedicine & Pharmacotherapy. 140: 111596).

Diosmetin was already reported to be effective against three RNA virus, enterovirus 71 (EV-A71) (Yin et al 2019. J Med Virol 91:1440-1447), hepatitis C virus (Rashed et al 2014. J App Pharm 6(1): 08-18) and coxsackie virus B3 (CVB3) (XiaoWen et al 2019. Chinese Journal of Pharmacology and Toxicology. 33 (7): 502-510).

Some studies have reported inhibitory activity of flavonoids against human coronavirus proteins (Russo et al 2020. Chemico-Biological Interactions 328:109211). There is also evidence that flavonoids have "pleiotropic" properties to counteract coronaviruses infection, acting on both viral and host cells. For instance, molecular docking screenings and *in vitro* assays showed that some flavonoids have potential to inhibit the host enzyme transmembrane protease serine 2 (TMPRSS2) and Furin (Rahman et al 2020. Molecules. 25: 2271). These enzymes facilitate viral particles entry into host cells, and were recently proposed as a promising COVID-19 treatment strategy (Palit et al 2021. Phytomedicine. 85: 153396). Eleven COVID-19 trials with flavonoid compounds are currently present in the database ClinicalTrial.gov.

The data contained herein demonstrates that the flavonoid diosmetin has the ability to efficiently suppress the viral propagation of SARS-CoV-2 in cell culture. This drug exerts an antiviral action against Coronavirus, through the inhibition of the RNases nsp15 and nsp14. Flavonoids are abundant in fruits and vegetables and therefore are considered as safe and non-toxic for human consumption.

### Terms and definitions

For purposes of interpreting this specification, the following definitions will apply and whenever appropriate, terms used in the singular will also include the plural and vice versa.

The terms "comprising," "having," "containing," and "including," and other similar forms, and grammatical equivalents thereof, as used herein, are intended to be equivalent in meaning and to be open ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictate otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a," "or," and "the" include plural referents unless the context clearly dictates otherwise.

### Detailed Description of the Invention

Administration of diosmetin is a clinically applicable treatment against human coronaviruses and their disease, including COVID-19 with post-exposure efficacy.

The inventors propose that targeting virus-specific RNA endonucleases confers significant advantages in hindering the amplification of the virus while posing little risk to the host. This is distinct from anti-viral agents, such as Remdesivir - the only FDA approved treatment against COVID-19. Remdesivir is an antiviral drug that belongs to the type of nucleoside analogue inhibitors (NAs) that stall the coronaviruses' RNA-dependent RNA polymerase. This drug has a conserved mode of inhibition across different viruses, where a single point mutation in the Ebolavirus polymerase leads to viral drug resistance. The use of NAs in coronaviruses is challenging due to the presence of the nsp14 ExoN proofreading activity. Nsp14 significantly attenuates the inhibitory effect of NA drugs that function through premature termination of viral genome replication. Indeed, WHO has issued a conditional recommendation against the use of remdesivir in hospitalized patients, regardless of disease severity, as there is no meaningful effect on mortality or on other important outcomes for patients, such as the need for mechanical ventilation or time to clinical improvement.

The inventors further propose that diosmetin can be used for the prevention or treatment of patients who have early to severe symptoms of COVID-19.

The inventors also propose that diosmetin, by lowering the viral load, may decrease the patient's chances of developing the persistent symptoms associated with long-COVID (fatigue, brain fog, headache, shortness of breath, joint and chest pain, etc.).

COVID-19 is characterized by an exuberant and systemic inflammatory response associated to the excessive production of inflammatory cytokines, such as tumor necrosis factor alpha (TNF-α) and interleukin-6 (IL-6). This event is known as "cytokine storm" and it is directly correlated with lung injury, septic shock, multi-organ failure, and subsequent mortality. High plasma levels of TNF-α and IL-6 have been associated with a poor prognosis of hospitalized patients with COVID-19 (Del Valle et al 2020. Nature Medicine 26: 1636-1643; Danlos et al 2021 European Journal of Cancer. 149: 102-104).

Targeting IL-6 receptors has shown efficacy in the treatment of severe COVID-19 patients (Guaraldi et al 2020. Lancet Rheumatol. 2:e474e84; Xu et al. 2020. Proc Natl Acad Sci USA. 117:10970e5; Sciascia et al. 2020. Clin Exp Rheumatol. 38: 529e32). In 6th July 2021, the World Health Organization (WHO) has updated its patient care guidelines to include IL-6 receptor blockers in severely ill patients with COVID-19, especially when administered together with corticosteroids. However, IL-6 receptor blockers remain inaccessible and unaffordable for the majority of the world (WHO).

Also, TNF-α blocking drugs were pointed out as potential therapies for patients with severe COVID-19 (Danlos et al 2021 European Journal of Cancer. 149: 102-104; Feldmann et al. 2020. Lancet. 395:1407e9).

Some studies have referred that diosmetin has an effect on cytokine secretion. The amount of TNF-α, IL-4 and IL-1β in skin lesion decreased in response to treatment with diosmetin (Lee et al 2020 Int Immunopharmacol. 89: 107046). Diosmetin also reduced IL-6 and TNF-α secreted levels in macrophages stimulated with lipopolysaccharides (LPS) (Mueller et al 2010. Food Chem. 122: 987-996). LPS are known to stimulate immune system cells, mainly macrophages, to activate signaling events that potentiate the production of inflammatory mediators, such as cytokines (Leyva-López et al 2016 Int J Mol Sci. 17(6): 921). Pretreatment with diosmetin also reduced levels of inflammatory proteins, such as IL-6, TNF-α and IL-1β in a murine model with cerulein-induced acute pancreatitis, and in mice with LPS and D-GalN-induced hepatitis (Yu et al. 2014. Int J Clin Exp Pathol. 7(5):2133-42; Yang et al 2017 Oncotarget. 8(19): 30723-30733).

COVID-19 can also cause a range of breathing problems, from mild to critical, with potential evolution to respiratory failure and acute respiratory distress syndrome. When people with asthma get respiratory infections, it can trigger their asthma symptoms. Effects of diosmetin were evaluated in a well-characterized murine model of allergic asthma. The authors reported that inflammation was remarkably relieved in the diosmetin group. Diosmetin was pointed as a potential and prominent anti-inflammatory agent for the management of airway diseases (Ge et al 2015. Acta Biochimica et Biophysica Sinica. 47(8): 604-611).

The *in vitro* experiments presented herein led the inventors to conclude that the optimal concentration for diosmetin is 45 µM (13.5 µg/ml). This optimal drug concentration was defined as the concentration of inhibitor that had the lowest toxic effect and simultaneously most protected cells from infection by SARS-CoV-2. 45 µM of diosmetin had an antiviral effect for 72h.

The effectiveness of diosmetin has been investigated against several other health conditions in mice, such as cognitive and memory impairment, colon cancer, atopic dermatitis, etc. (Saghaei et al 2020. Evidence-Based Complementary and Alternative Medicine Article ID 5725361; Koosha et al 2019. Molecules. 24(14): 2522). Diosmetin did not demonstrate any signs of toxicity or mortality in treated ICR mice, during 14 days of observation, after oral administration of a single dose of 300 mg/kg and 2000 mg/kg (Koosha et al 2019. Molecules. 24(14): 2522).

Cova et al showed the total absence of urinary elimination for diosmetin, while minor metabolites are eliminated in the urine (Cova et al 1992. Int J Clin Pharmacol Ther Toxicol 30(1):29-33). Diosmetin is partly excreted in bile as the glucuronide and sulphate. Diosmetin did not appear to affect various parameters of liver function (Perego et al 1993. 23(12): 1345-1352).

Here, the inventors report the antiviral capacity of diosmetin against SARS-CoV-2. Its ability to inhibit excessive production of pro-inflammatory cytokines could also suppress systemic inflammatory responses, attenuating disease progression, and improving prognosis of severe COVID-19.

### General treatment criteria

The SARS-CoV-2 positive patient to be treated with diosmetin presents at the hospital with the most common COVID-19 symptoms, including
- Respiratory illness symptoms (sore throat, dry cough, shortness of breath, chest pain)
- High temperature
- Sudden loss of sense of smell and/or taste

Possibly other symptoms are also present, such as
- Headache
- General weakness, unwellness
- Aching muscles
- Gastrointestinal symptoms (nausea, vomiting, diarrhea, stomachache)
- Head cold
- Skin rash
- Sore eyes

### High dosage (more than twice daily)

Patients to whom one or more of the risk criteria apply will receive the high dose treatment:
- age >60 years;
- high blood pressure;
- cardiovascular disease;
- diabetes (particularly diabetes mellitus type II);
- chronic respiratory diseases;
- obesity class III (morbid, BMI greater than or equal to 40 kg/m2).

### TREATMENT DURATION

Treatment should last for 10-20 days with intermittent presentation at the hospital for blood tests, including immune cell analyses, cytokines and oxygenation levels, as well as viral titer determination.

The data provided in here demonstrate that diosmetin exerts an antiviral effect on SARS-CoV-2. Diosmetin inhibits SARS-CoV-2 propagation by non-competitive inhibition of RNA nucleases nsp14 (a 3'-5' exoribonuclease and guanine-N7-methyl-transferase acting in coronavirus replication and transcription) and nsp15 (an endoribonuclease that preferentially cleaves 3' of U residues avoiding immune system activation) (Saramago, M., et al. The FEBS Journal 2021, 288: 5130-5147; Saramago M., et al. Microorganisms 2022, 210(2):342022). Indeed, both enzymes are essential and were identified as major interferon antagonists from SARS-CoV-2 (Yuen et al CK Emerg Microbes Infect. 2020 9:1418-1428). Moreover, diosmetin with its ability to inhibit excessive production of pro-inflammatory cytokines may have beneficial and synergistic effects on SARS-CoV-2 replication / dissemination, and in COVID-19 inflammatory pathogenesis / disease progression.

### Medical treatment, Dosage Forms

Within the scope of the present invention is a method or treating COVID-19 in a patient in need thereof, comprising administering to the patient diosmetin according to the above description.

Similarly, a dosage form for the treatment of human coronavirus infection, particularly of infection with SARS-CoV-2 (COVID-19), is provided, comprising diosmetin according to any of the above aspects or embodiments of the invention.

### Pharmaceutical Compositions and Administration

Another aspect of the invention relates to a pharmaceutical composition comprising diosmetin, and a pharmaceutically acceptable carrier.

In certain embodiments of the invention, the compound of the present invention is typically formulated into pharmaceutical dosage forms to provide an easily controllable dosage of the drug and to give the patient an elegant and easily handleable product.

The invention is further illustrated by the following examples and figures, from which further embodiments and advantages can be drawn. These examples are meant to illustrate the invention.

### Description of the Figures

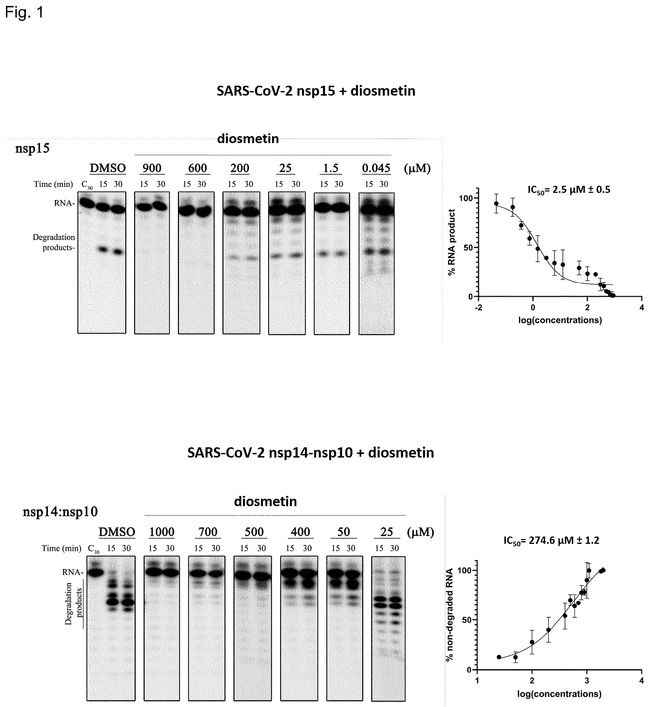

Fig. 1 shows the degradation of RNA product based on activity of both nsp15 or nsp14 in response to varying concentrations of diosmetin. A synthetic 22-mer oligoribonucleotide (H4, see Saramago et al. FEBS J. 288(17) 2021, 5130-5147) was used as a substrate for nsp14 and a 16-mer oligoribonucleotide (Ortiz-Alcantara et al., Virus Adaption and Treatment, 2010; Saramago, Microorganisms 2022, ibid.) for nsp15. Both RNAs were labelled at their 5' end with [³²P]-γ-ATP and T4 Polynucleotide Kinase (Ambion) in a standard reaction. MicroSpin G-50 columns (Cytiva) were used to remove excess [³²P]-γ-ATP. The synthetic RNAs were resuspended in 10 mM of Tris-HCl pH 8.0 and incubated 10 min at 80°C followed by 45 min at 37°C. Different concentrations (µM) of diosmetin were incubated, 7 minutes at room temperature, with 300 nM of nsp15 or with 40 nM-160 nM of nsp14-nsp10 proteins, before initiating the *in vitro* assay through the addition of the P³²-RNA substrate (50 nM). The activity assays were performed in a final volume of 12 µl containing the activity buffer 50 mM HEPES pH 7.4, 1 mM DTT, 5 mM MgCl₂ in case of the nsp14-nsp10 complex, and 25 mM HEPES pH 7.4, 50 mM NaCl, 1 mM DTT, 10 mM NaHPO₄, 5 mM MnCl₂ for nsp15. The reactions were incubated at 37°C, and then aliquots of 4 µl were withdrawn at the time-points indicated in the respective figures. The reactions were stopped by adding a gelloading buffer containing 80% (v/v) formamide and 10 mM EDTA. Diosmetin was solubilized and diluted in DMSO, whose concentration was kept constant and corresponds to the maximum amount of diosmetin tested in the assay. Reaction controls were performed with the RNAs (without the enzymes), with either activity buffer or buffer supplemented with DMSO. Controls were incubated in the same conditions during the assay. Reaction products were resolved in a 20% denaturant polyacrylamide gel (7M urea). Signals were visualized by PhosphorImaging (TLA-5100 Series, Fuji). All the experiments were performed at least in triplicate. Inhibition profile curves were calculated by quantification of gel-based RNase assays. Data were plotted against log₁₀ of [diosmetin] and dose response curves were generated using non-linear regression (GraphPad Prism 8 software). At least three biological replicates were used for each concentration.

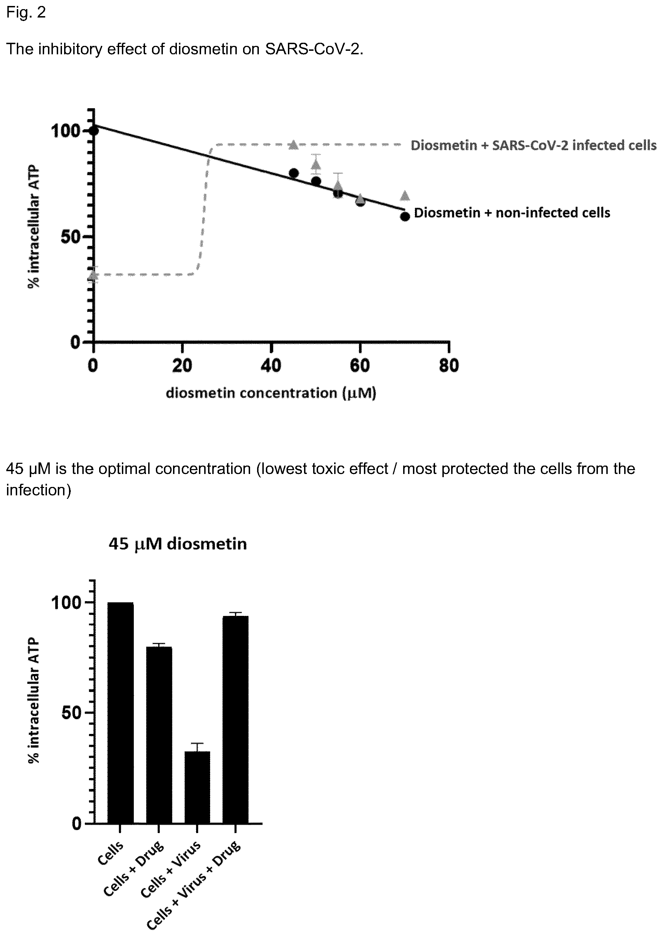

Fig. 2 shows bioluminescence of cells either control or infected with SARS-CoV-2 and untreated or treated with diosmetin. The bioluminescence signal corresponds directly to the amounts of intracellular ATP and reflects viability of cells. The inhibitory effect of diosmetin on SARS-CoV-2 multiplication in Vero E6 cells was evaluated. Briefly, Vero E6 cells were harvested and suspended in MEM medium supplemented with 10% fetal bovine serum (FBS), penicillin (100 U/ml) and streptomycin (100 mg/ml). Different concentrations of diosmetin were mixed with 3 x 10⁴ Vero E6 cells and platted into 96-well cell culture plates (100 µl/well). SARS-CoV-2 (30 PFUs / 25 µl) was then added to Vero E6 cells and the plates were incubated at 37°C in a 5% CO2 atmosphere for 72h. After that period plates were examined for the presence or absence of cytopathic effect (CPE) under a light microscope. In addition, the viability of cells in culture was assessed by measuring intracellular ATP levels, using a luminescent assay as described above (CellTiter-Glo^{®} Luminescent Cell Viability Assay). The luminescent signal is proportional to the amount of ATP present and the optimal drug concentration was chosen as the concentration of drug that most protected cells from infection by SARS-CoV-2, having simultaneously lower effect in cell viability in the absence of virus. Cellular ATP levels in the presence of various concentrations of diosmetin and the ability of the drug to recover cells from SARS-CoV-2 infection were plotted using GraphPad Prism 8 software.

### Examples

### Example 1

AutoDock Vina (Trott and Olson, Journal of Computational Chemistry, 2010 31(2), 455-461) was used to analyze the interaction of several sets of compounds with SARS-CoV-2 nsp14 and nsp15. Drugs were retrieved from Drugbank (doi: 10.1093/nar/gkx1037) datasets: FDA Approved, Experimental, Investigational and Nutraceutical. Ligand files were prepared for docking using AutoDockTools 1.5.6 utilities by assigning gasteiger charges, AD4 atom types and rotable bonds. The protein structures found in PDB IDs 6VWW (doi: 10.2210/pdb6VWW/pdb and doi: 10.1002/pro.3873) and 6WXC (doi: 10.2210/pdb6WXC/pdb and doi: 10.1038/s42003-021-01735-9) were used for the nsp15 screenings. All non-proteic compounds were removed except for the phosphate ion that was present in the active site of 6WXC. Since the structure of SARS-CoV-2 nsp14 RNase was not available at the time the inventors performed the dockings, and given the very high identity with the homologous protein of SARS-CoV-1, they used a homology-model previously built in-house (Saramago, M. ibid.). However, the structure already available corroborates their model (Moeller, NH et al PNAS 2022, 119(9): e2106379119). All proteins were prepared for docking calculations using AutoDockTools by computing gasteiger charges and assigning AD4 atom types. No flexibility was taken into account in the receptor structures.

Plasmids expressing nsp10, nsp14 and nsp15 were obtained as described by Saramago, M ibid. Nsp10, nsp14 and nsp5 were expressed and purified as previously described (Saramago M., et al. Microorganisms 2022, 210(2):342022).

Synthetic RNAs already described in the literature were used and labeled at their 5' end with [γ-³²P]-ATP (Bouvet et al PNAS 2012: 109: 9372-9377; Ortiz-Alcantara et al Virus Adaptation and Treatment 2010:2 125-133; Saramago, M. ibid.). In the assays with nsp14-nsp10 protein complex, the H4 RNA was used. It consists in a 22-nucleotide (nt) long RNA with a 5' singlestranded (ss) tail and a 3'-end engaged in a stable duplex structure (Saramago, M. ibid.). The nsp14 ExoN activity was then evaluated by analyzing the hydrolysis of the H4 RNA 5'-end radiolabeled with a ratio nsp14-nsp10 of 1:4 (Saramago, M. ibid.). The reaction products from this reaction range between 17- to 20-nts in length, which corresponds to cleavage from the 3'-end of the RNA duplex region (Saramago, M. ibid.). To test the activity of nsp15, a synthetic RNA previously reported in Ortiz-Alcantara et al. 2010 was used. This substrate is a 16-nt long RNA with a 5'-end with two ss-nucleotides followed by a stem-lop structure and a 3'-ss tail (Ortiz-Alcantara et al Virus Adaptation and Treatment 2010:2 125-133). This RNA contains a unique U residue where the enzyme cleaves, generating two breakdown products. Since the RNA molecule is labelled at its 5'end, only the upstream degradation product is visible in the gel.

In the reactions performed, the RNA substrates and enzymes concentrations were kept constant. Reaction controls were performed in the absence of the enzymes, with buffer supplemented with DMSO, since this solvent was used to solubilize the drug. Diosmetin has an inhibitory effect on the activity of both viral RNases. To evaluate this effect over SARS-CoV-2 nsp15, a range of concentrations between 900 and 0.045 µM was tested (only some of the concentrations are represented in the figure). The inventors demonstrate that diosmetin is able to inhibit SARS-CoV-2 nsp15 activity with a IC₅₀ of 2.5 ± 0.5 µM. Regarding the nsp14-nsp10 protein complex, a range of concentrations between 1,000 and 25 µM was tested. Diosmetin is also able to block the exoribonucleolytic activity of nsp14 with an IC₅₀ of 274.6 ± 1.2 µM.

The results are shown in Figure 1.

### Example 2

Virus isolation was performed in biosafety level 3 (BSL3) facilities at INIAV-Laboratory of Virology. SARS-CoV-2 isolate (ICV1006) was obtained from a Portuguese COVID19 patient. The production of virus stock was accomplished in Vero E6 cells (African green monkey kidney cells, catalog no.ATCC CRL-1586) maintained in Eagle's minimum essential medium (MEM) supplemented with 10 % fetal bovine serum (FBS) and penicillin (100 U/ml) and streptomycin (100 mg/ml), at 37°C in a 5% carbon dioxide atmosphere. After three times freeze-thaw cycles the cultures were clarified at 1500 X g for 15 min at 5°C. The clarified supernatant was filtered through a 0.22 µm filter, aliquoted and kept at -80° C until used. The infectivity titer of the viral stock was determined by a standard plaque assay.

The potential cellular toxicity of diosmetin in Vero E6 cells was determined by measuring cellular ATP levels in the presence of various concentrations of the drug (45, 50, 55, 60 and 70 µM) (Figure 2). To perform this, cytotoxicity in cell wells was measured 72 h after treatment with diosmetin, using the CellTiter-Glo luminescent cell viability assay kit (Promega) according to the manufacturer's instructions. Briefly, a total of 50 µl of medium were removed and discarded from each well and 50 µl of reconstituted CellTiter-Glo reagent were added. After 2 min agitation to promote cell lysis, the mixtures were transferred to an opaque plate and incubated for 10 minutes to stabilize the luminescent signal that was then measured in a Luminoscan Ascent Luminometer (Labsystems). The maximum nontoxic concentration was defined as the concentration of inhibitor that had minor or no effect on cellular ATP levels.

Diosmetin was also evaluated in cell infection assays with SARS-CoV-2. To accomplish this goal, Vero E6 cells were incubated with increasing concentrations of diosmetin in the presence of 30 pfu SARS-CoV-2. The virus was isolated from a Portuguese COVID-19 positive patient sample provided by the reference National Institute of Health (INSA, Portugal). The effect of diosmetin on SARS-CoV-2 replication was investigated in plaque formation assays and by measuring intracellular ATP levels in viable cells using CellTiter-Glo assay (Promega).

As a control, ATP levels were measured on Vero E6 cells, and that value was considered 100% (Figure2). ATP was also measured on cells infected with SARS-CoV-2. Obviously, there was a drastic decrease on the levels of ATP upon infection, indicating that there are less metabolically active cells. As already mentioned, in order to determine the toxicity of the drug, Vero E6 cells were incubated with different concentrations of diosmetin (45, 50, 55, 60 and 70 µM). The optimal drug concentration was defined as the concentration of diosmetin that had the lowest or no effect on cellular ATP levels in the absence of virus, and simultaneously most protected cells from infection by SARS-CoV-2. These experiments led to conclude that the optimal concentration for diosmetin is 45 µM (13.5 µg/ml). When 45 µM of this drug were added to the Vero E6 cells infected with SARS-CoV-2, there is a total rescue of the cells from the viral infection.

The results are shown in Figure 2.

### Example 3

Sequence alignment of nsp15 from MERS-CoV, SARS-CoV-1, SARS-CoV-2 Omicron BA.4, SARS-CoV-2, SARS-CoV-2 Alpha, SARS-CoV-2 Beta, SARS-CoV-2 Gamma, SARS-CoV-2 Omicron BA.1, SARS-CoV-2 Delta. Important residues for nsp15 RNase activity, specificity to degrade U residues and protein hexamerization, include N164, H235, H250, K290, S294, T341 and Y343 - in SARS-CoV-2 (Saramago M., et al. Microorganisms 2022, 210(2):342022). These residues are fully conserved in SARS-CoV-2 and its variants of concern, SARS-CoV-1 and MERS. In fact, nsp15 is fully conserved among the emerging variants of concern with no mutations recorded. Additionally, SARS-CoV-2 nsp15 shares 88.7% sequence identity and 94.8% of similarity with the SARS-CoV-1 homolog, and 50.1% sequence identity, and 63.9% similarity with the homolog from MERS-CoV. The same important residues maintain their conservation in SARS-CoV-1 and MERS-CoV.

A sequence alignment of nsp14 from MERS-CoV, SARS-CoV-1, SARS-CoV-2 Omicron BA.4, SARS-CoV-2, SARS-CoV-2 Alpha, SARS-CoV-2 Beta, SARS-CoV-2 Gamma, SARS-CoV-2 Omicron BA.1, and SARS-CoV-2 Delta can be performed. Nsp14 belongs to the superfamily of DEDD exonucleases, and these active site residues are recited in the preceding paragraph. These amino acids are distributed over three canonical motifs: motif I (D90/E92), motif II (D243) and motif III (D273) - numbering according to SARS-CoV-2. These important catalytic residues are fully conserved in SARS-CoV-2 and its variants of concern, SARS-CoV-1 and MERS-CoV (Saramago, M., et al. The FEBS Journal 2021, 288: 5130-5147). Indeed, nsp14 is fully conserved among the emerging variants of concern with only one mutation detected in the Omicron variants, the I42V. However, this amino acid is not located at the active center or at the interaction surface with the nsp10 protein with which nsp14 interacts to form a heterodimer. So, this mutation has no biological meaning. This information is supported by protein modelling data on the omicron nsp14 (data not shown). Additionally, SARS-CoV-2 nsp14 has high similarity with nsp14 from SARS-CoV-1 (95% of sequence identity and 99.1% of sequence similarity). Less conservation with the MERS-CoV amino acid sequence is found, but the crucial residues for the RNase activity of nsp14 maintain their conservation in SARS-CoV-1 and MERS-CoV.

This information confirms that diosmetin is equally effective in inhibiting the activity of the RNases nsp15 and nsp14 of the SARS-CoV-2 variants and thus exerting an antiviral effect. And most likely, it will also be effective against other human coronaviruses, namely SARS-CoV-1 and MERS-CoV.

## Claims

1. Diosmetin for use in the treatment of a human coronavirus infection.

2. Diosmetin for use according to claim 1, wherein the infection is an infection by SARS-CoV-2.

3. Diosmetin for use according to claim 1, wherein the infection is an infection by an emerging variant of SARS-CoV-2, particularly a variant selected from the group consisting of SARS-CoV-2 alpha, SARS-CoV-2 beta, SARS-CoV-2 gamma, SARS-CoV-2 delta, SARS-CoV-2 omicron.

4. Diosmetin for use according to claim 1, 2 or 3, wherein the drug is administered at a dose that leads to a plasma level of 1-100 µmol/L.

5. Diosmetin for use according to claim 1, 2 or 3, wherein the drug is administered at a dose that leads to a plasma level of 5 µmol/L to 50 µmol/L.

6. Diosmetin for use according to claims 3, 4 or 5, wherein the drug is administered once daily / twice daily by oral administration form.

7. Diosmetin for use according to claims 4 or 5, wherein the drug is administered by systemic administration.

8. Diosmetin for use according to any one of the preceding claims 2 to 7, wherein the drug is administered to a patient having been diagnosed with COVID-19 in the 28 hours preceding the administration.

9. Diosmetin for use according to any of the preceding claims 2 to 8, wherein the drug is administered to a patient showing mild symptoms of COVID-19, wherein the patient is normoxic, particularly wherein the blood oxygenation level is equal or larger than 90%.

10. Diosmetin for use according to any of the preceding claims 2 to 8, wherein the drug is administered to a patient showing moderate to severe symptoms of COVID-19, wherein the patient is **characterized by** one or more of the following risk factors:
- age >60 years;
- high blood pressure;
- cardiovascular disease;
- diabetes (particularly diabetes mellitus type II);
- chronic respiratory diseases;
- obesity class III (morbid, BMI greater than or equal to 40 kg/m2) and /or
the patient's blood oxygenation level is below 90%.

## Patentansprüche

1. Diosmetin zur Verwendung bei der Behandlung von einer Infektion durch das menschliche Coronavirus.

2. Diosmetin zur Verwendung gemäß Anspruch 1, wobei die Infektion eine Infektion durch SARS-CoV-2 ist.

3. Diosmetin zur Verwendung gemäß Anspruch 1, wobei die Infektion eine Infektion durch eine neu auftretende Variante von SARS-CoV-2 ist, insbesondere eine Variante, die aus der Gruppe ausgewählt ist, die aus SARS-CoV-2 Alpha, SARS-CoV-2 Beta, SARS-CoV-2 Gamma, SARS-CoV-2 Delta und SARS-CoV-2 Omikron besteht.

4. Diosmetin zur Verwendung gemäß Anspruch 1, 2 oder 3, wobei das Arzneimittel in einer Dosis verabreicht wird, die zu einer Plasmakonzentration von 1-100 µmol/L führt.

5. Diosmetin zur Verwendung gemäß Anspruch 1, 2 oder 3, wobei das Arzneimittel in einer Dosis verabreicht wird, die zu einer Plasmakonzentration von 5 µmol/L bis 50 µmol/L führt.

6. Diosmetin zur Verwendung gemäß den Ansprüchen 3, 4 oder 5, wobei das Arzneimittel einmal täglich/zweimal täglich in oraler Darreichungsform verabreicht wird.

7. Diosmetin zur Verwendung gemäß den Ansprüchen 4 oder 5, wobei das Arzneimittel durch systemische Verabreichung verabreicht wird.

8. Diosmetin zur Verwendung gemäß einem der vorstehenden Ansprüche 2 bis 7, wobei das Arzneimittel einem Patienten verabreicht wird, bei dem in den 28 Stunden vor der Verabreichung COVID-19 diagnostiziert wurde.

9. Diosmetin zur Verwendung gemäß einem der vorstehenden Ansprüche 2 bis 8, wobei das Arzneimittel einem Patienten mit leichten Symptomen von COVID-19 verabreicht wird, wobei der Patient normoxisch ist, insbesondere wobei der Blutsauerstoffgehalt gleich oder größer als 90 % ist.

10. Diosmetin zur Verwendung gemäß einem der vorstehenden Ansprüche 2 bis 8, wobei das Arzneimittel einem Patienten mit mittelschweren bis schweren Symptomen von COVID-19 verabreicht wird, wobei der Patient durch einen oder mehrere der folgenden Risikofaktoren gekennzeichnet ist:
- Alter > 60 Jahre;
- Bluthochdruck;
- Kardiovaskuläre Erkrankung;
- Diabetes (insbesondere Diabetes mellitus Typ II);
- chronische Atemwegserkrankungen;
- Adipositasgrad III (morbid, BMI größer oder gleich 40 kg/m2) und/oder
der Blutsauerstoffgehalt des Patienten liegt unter 90 %.

## Revendications

1. Diosmétine destinée à être utilisée dans le traitement d'une infection par le coronavirus humain.

2. Diosmétine destinée à être utilisée selon la revendication 1, dans laquelle l'infection est une infection par SARS-CoV-2.

3. Diosmétine destinée à être utilisée selon la revendication 1, dans laquelle l'infection est une infection par un variant émergent de SARS-CoV-2, notamment un variant sélectionné parmi le groupe constitué de SARS-CoV-2 alpha, SARS-CoV-2 bêta, SARS-CoV-2 gamma, SARS-CoV-2 delta, SARS-CoV-2 omicron.

4. Diosmétine destinée à être utilisée selon la revendication 1, 2 ou 3, dans laquelle le médicament est administré à une dose qui conduit à un niveau plasmatique de 1 à 100 µmol/l.

5. Diosmétine destinée à être utilisée selon la revendication 1, 2 ou 3, dans laquelle le médicament est administré à une dose qui conduit à un niveau plasmatique de 5 µmol/l à 50 µmol/l.

6. Diosmétine destinée à être utilisée selon la revendication 3, 4 ou 5, dans laquelle le médicament est administré une fois par jour / deux fois par jour par forme d'administration orale.

7. Diosmétine destinée à être utilisée selon la revendication 4 ou 5, dans laquelle le médicament est administré par administration systémique.

8. Diosmétine destinée à être utilisée selon l'une quelconque des revendications précédentes 2 à 7, dans laquelle le médicament est administré à un patient ayant été diagnostiqué du COVID-19 dans les 28 heures précédant l'administration.

9. Diosmétine destinée à être utilisée selon l'une quelconque des revendications précédentes 2 à 8, dans laquelle le médicament est administré à un patient présentant de légers symptômes du COVID-19, dans laquelle le patient est normoxique, notamment dans laquelle le niveau d'oxygénation sanguine est supérieur ou égal à 90 %.

10. Diosmétine destinée à être utilisée selon l'une quelconque des revendications précédentes 2 à 8, dans laquelle le médicament est administré à un patient présentant des symptômes modérés à graves du COVID-19, dans laquelle le patient est **caractérisé par** un ou plusieurs des facteurs de risque suivants :
- âge > 60 ans ;
- tension élevée ;
- maladie cardiovasculaire ;
- diabète (notamment diabète sucré de type II) ;
- maladies respiratoires chroniques ;
- obésité de classe III (morbide, IMC supérieur ou égal à 40 kg/m²) et/ou
le niveau d'oxygénation sanguine du patient est en dessous de 90 %.
